# EUROPEAN PATENT APPLICATION

(11) **EP 4 687 154 A1**
(43) Date of publication of application: **04.02.2026**
(21) Application number: 24315363.2
(22) Date of filing: 30.07.2024
(51) Int. Cl.: G16H 50/50

(54) **A COMPUTER-IMPLEMENTED METHOD FOR TRAINING AT LEAST ONE DEPLOYMENT MODEL, A COMPUTER PROGRAM, A NON-TRANSITORY COMPUTER-READABLE MEDIUM, A TRAINED MACHINE-LEARNING MODEL, A COMPUTER IMPLEMENTED METHOD FOR PREDICTING A DEPLOYMENT OF A HEART VALVE PROSTHESIS, A MEDICAL APPARATUS, AND A METHOD FOR DEPLOYING A HEART VALVE PROSTHESIS**

(71) Applicant: Caranx Medical, 75013 Paris (FR)
(72) Inventor: MUKHINA, Ekaterina, 06000 Nice (FR); BERTHET-RAYNE, Pierre, 06800 Cagnes-sur-Mer (FR); SCHEGG, Pierre, 0600 Nice (FR); SMITS, Jonas Victor Harmen, 3360 Bierbeek (BE); PRZYBYLSKI, Flavie, 06800 Cagnes sur Mer (FR); WEI, Wen, 06410 Biot (FR)
(74) Representative: Hepp Wenger Ryffel AG

(57) **Abstract**

A computer-implemented method for training at least one deployment model to predict a deployment of a heart valve prosthesis assembly (2, 3, 4) comprising the steps of receiving, by one or more processors, a baseline image sequence. The baseline image sequence comprises images which include the heart valve prosthesis assembly (2, 3, 4) within a native anatomic structure (9) in at least two different deployment states. The method includes extracting at least one heart valve prosthesis assembly characteristic of the heart valve prosthesis assembly (2, 3, 4) from the baseline image sequence. The method optionally includes extracting at least one anatomic characteristic of the native anatomic structure (9) from the baseline image sequence. The method comprises training the at least one deployment model for the heart valve prosthesis assembly (2, 3, 4) based on the heart valve prosthesis assembly characteristic, and optionally the anatomic characteristic. The deployment model is configured for predicting an intra-operative future deployment state of a heart valve prosthesis assembly (2', 3', 4') based on an intra-operative image sequence comprising the heart valve prosthesis assembly (2', 3', 4') in a current deployment state.

## Description

The invention relates to a computer-implemented method for training at least one deployment model to predict a deployment of a heart valve prosthesis assembly, a non-transitory computer-readable medium, a trained machine-learning model, a computer implemented method for predicting a deployment of a heart valve prosthesis, a medical apparatus for positioning and deploying a heart valve prosthesis assembly, a method for using an intra-operative future deployment of a heart valve prosthesis assembly, and a method for positioning and deploying a heart valve prosthesis.

Specialized minimally invasive surgical heart valve procedures often need to be performed by highly trained operators, in particular a plurality of operators simultaneously, to enable accurate and consistent pose adjustment and/or deployment of medical instruments/implants, in particular in view of movement of the medical instrument in an aortic-ventricular direction. This adjustment and/or deployment of heart valve prostheses, delivery systems, guidewires, and catheters can be challenging due to a difficult to predict deployment behavior during minimally invasive procedures, in particular due to the behavior being influenced by multi variate factors. This challenge is further compounded by human sensory limitations during the transcatheter procedure and limited operational capabilities, which inhibit the ability to perceive and effectively manage the intricate dynamics of these multivariate factors. In addition to the behavior being difficult to predict due to inherent complexity, the clinician is limited by medical imaging devices only providing an indirect assessment of the medical instrument and a mere approximation of the actual biological complexities.

In particular, transcatheter aortic valve implantation/replacement (TAVI/TAVR) which is considered to be a safe and effective procedure, e.g. for the treatment of aortic stenosis of patients not eligible for conventional surgical treatment, necessitates predicting the intra-operative behavior of a heart valve prosthesis assembly, in particular a heart valve prosthesis and/or a deployment instrument of the assembly, based on image sequences for different patients and heart valve prosthesis assemblies. Failure to predict the behavior of the heart valve prosthesis/deployment instrument and/or delivery instrument can lead to adverse procedural outcomes such as malpositioning in 15.3% of cases and even ostial coronary obstruction in 3.5% of the cases as described by Dvir et al. in "Transcatheter Aortic Valve Replacement for Degenerative Bioprosthetic Surgical Valves" (Circulation, vol. 126, no. 19, pp. 2335-2344, November 2012, DOI: 10.1161/circulationaha.112.104505).

As demonstrated by Esmailie et al. (2022) in their study on the biomechanics of transcatheter aortic valve replacement complications and computational predictive modeling, the computational modelling of the biomechanical behavior during TAVR can be applied to assess risk of tissue rupture, coronary obstruction, paravalvular leak, valve thrombosis (Structural Heart, vol. 6, no. 2, Article 100032, DOI: 10.1016/j.shj.2022.100032).

Nonetheless, a clinician/a robotic controller lacks information on how to accurately anticipate movement and behaviour of a heart valve prosthesis assembly during deployment and, in particular where to position and how to orient the heart valve prosthesis during deployment such that an optimal position can be achieved with respect to the patient anatomy and/or the deployment instrument after deployment.

The prior art further lacks a precise computer-implemented method and a medical apparatus enabling a prediction of the deployment behavior of a prosthetic heart valve throughout deployment within a native anatomy of a patient based on medical image data.

In particular, the prior art lacks a predictive model which enables accurate predictions of the position/orientation of a heart valve prosthesis during and after deployment which is reliable for different heart valve prosthesis types, anatomy types, and/or sizes. If available, such a prediction would allow to infer an accurate and reliable future deployment state, in particular a target deployment position/orientation, of the heart valve based on a current deployment position, in particular a current/prior position/orientation, in the image data in which the prosthetic heart valve is not or at least not fully deployed yet.

It is an object of the present invention to overcome these and other disadvantages of the prior art. The present invention shall provide computer-implemented methods and/or a medical apparatus which solve the above-mentioned technical problems and enable reliable predictions of the behavior of a heart valve prosthesis assembly to facilitate positioning the heart valve prosthesis assembly such that the heart valve prosthesis of the heart valve prosthesis assembly can be positioned at its optimal location within the heart valve despite being affected by multivariate factors. In particular, the present invention shall counteract at least one of the following adverse effects on the position/orientation of a heart valve prosthesis during deployment: eccentric mounting on a balloon catheter, valve shift with respect to a balloon of the balloon catheter during inflation, balloon shift with respect to the anatomy, balloon inflation variability, interaction with vascular plaque, delivery system shift, and/or obstruction of view.

The computer-implemented method is for training at least one deployment model to predict a deployment of a heart valve prosthesis assembly.

The method comprises receiving, by one or more processors, a baseline image sequence which comprises images which include the heart valve prosthesis assembly within a native anatomic structure in at least two different deployment states. The method includes extracting at least one heart valve prosthesis assembly characteristic of the heart valve prosthesis assembly from the baseline image sequence. The method optionally includes extracting at least one anatomic characteristic of the native anatomic structure from the baseline image sequence. The method comprises training and/or computational modelling of the at least one deployment model for the heart valve prosthesis assembly based on the heart valve prosthesis assembly characteristic, and optionally the anatomic characteristic. The deployment model is configured for predicting an intra-operative future deployment state of an intra-operative heart valve prosthesis assembly based on an intra-operative image sequence comprising the intra-operative heart valve prosthesis assembly in a current deployment state.

Alternatively to training, the method may comprise computational modelling, e.g. using a statistical or biomechanical model, of the at least one deployment model for the heart valve prosthesis assembly based on the heart valve prosthesis assembly characteristic, and optionally the anatomic characteristic.

The heart valve prosthesis assembly may comprise or consist of at least one of a heart valve prosthesis, and optionally a deployment instrument for deploying the heart valve prosthesis and/or a navigation instrument for navigating the heart valve prosthesis. The heart valve prosthesis assembly characteristic may be determined based on at least one of the heart valve prosthesis, the medical deployment instrument, and the medical navigation instrument.

The heart valve prosthesis assembly characteristic and/or the anatomic characteristic may be extracted using a computer vision algorithm, by providing or generating labelled baseline image data, and/or processing of the extracted/labelled data.

The first and/or the intra-operative current deployment state may be a partially deployed or non-deployed state of the heart valve prosthesis assembly. The second deployment state and/or the intra-operative future deployment state may be a partially or fully deployed state.

The respective deployment states may comprise or consist of a spatial position, an orientation, and/or a deployment configuration of the heart valve prosthesis assembly, in particular of the heart valve prosthesis, the deployment instrument, and/or the navigation instrument. The deployment configuration may be a specific inflation configuration of a deployment instrument such as a balloon catheter.

The native anatomic structure may comprise or consist of the aortic heart valve, the mitral valve, the native aortic valve annulus, the coronary arteries, the aortic leaflets, the coronary cusps, the left ventricle, the aortic root, the sinotubular junction, the aortic arch, and the atrioventricular node.

The fluoroscopy image sequence may comprise or consist of a fluoroscopy image sequence and/or a computer tomography image sequence. The intra-operative fluoroscopy image sequence may comprise or consist of an intra-operative fluoroscopy image sequence and/or an intra-operative computer tomography image sequence.

The heart valve prosthesis may be a heart valve prosthesis which is deployable by a balloon catheter or may be a self-deployable heart valve prosthesis.

The baseline image sequence in the context of this application relates to a non-intra-operative image sequence, e.g. a baseline image sequence acquired prior during a surgical procedure. A plurality of baseline image sequences from different surgical procedures may be received by one or more processors.

The baseline image sequence may consist only of a baseline image sequence comprising the heart valve prosthesis assembly being deployed within a native anatomic structure, i.e. being converted from a first non-deployed state to a second fully deployed state. By preprocessing the baseline image sequence to only include the valve prosthesis deployment, e.g. only the process of balloon inflation of the balloon catheter, the prediction of the future deployment state may be optimized. This further facilitates providing a specialized deployment model for predicting the deployment and simplifies an implementation of the model.

The intra-operative image sequence may be acquired during a medical procedure such as TAVI/TAVR or mitral valve implantation/replacement. All computer-implemented method steps to train the deployment model are in particular carried out prior to carrying out the medical procedure, i.e. preoperatively and not intra-operatively. Such a pre-trained model can operate on local storage independently of remote servers or cloud integration.

Training the at least one deployment model based on the heart valve prosthesis assembly characteristic, and optionally the anatomic characteristic, may comprise applying a clustering algorithm to the baseline image sequence and/or the heart valve prosthesis assembly characteristic, and optionally the anatomic characteristic. Additionally or alternatively, the processing may comprise applying a principal component analysis (PCA) to this data, preferably after using the clustering algorithm. This may be carried out prior to generating the at least one deployment model. By using a clustering analysis, a plurality of deployment models may be tuned for different deployment scenarios based on similarity with respect to each other, e.g. using similar types of heart valve prostheses or the heart valve prostheses being deployed in similar anatomic structures.

The heart valve prosthesis assembly characteristic may be selected from (i) a specific heart valve prosthesis type, (ii) a heart valve prosthesis dimension, in particular at least one radial dimension and/or a longitudinal dimension, (iii) a point-to-point or line-to-line distance between the heart valve prosthesis and the anatomic structure or a medical instrument, and/or (iv) a deployment dynamics of deploying the heart valve prosthesis, in particular a relation between the time for deployment, a velocity of deployment, and/or an acceleration of deployment. The heart valve prosthesis assembly characteristic may be (v) a metric indicative of a displacement of a geometric center of the heart valve prosthesis relative to a deployment instrument or the anatomic structure in an axial direction of the heart valve prosthesis assembly.

Selecting the heart valve prosthesis assembly characteristic from at least one of (i) - (ii) enables generating a more versatile deployment model and provides an optimized solution for specific heart valve prosthesis types/dimensions which enhances procedural accuracy and safety. Selecting the heart valve prosthesis assembly characteristic of (iii) provides a reliable, simple metric of relating the heart valve prosthesis to the anatomic structure or the medical instrument and, thus, reduces complexity for predicting the deployment. Selecting the heart valve prosthesis assembly characteristic of (iv) enables to indirectly take into account different inherent spatiotemporal relations which influence the deployment dynamics of the heart valve prosthesis, such as for example a higher velocity flow due to a restriction of the vasculature by a balloon catheter at later stages of the deployment which may result in displacement.

The heart valve prosthesis assembly characteristic according to (v) enables the deployment model to predict the relative displacement of the heart valve prosthesis with respect to the deployment instrument, in particular the balloon catheter, to compensate for asymmetric/non-uniform deployment. The heart valve prosthesis assembly characteristic visible in the baseline image data may comprise at least distance/metric indicative of the asymmetric/non-uniform deployment of the heart valve prosthesis assembly.

This may be carried out based on tracking the heart valve prosthesis, in particular the geometric center of the heart valve prosthesis, and a deployment instrument, in particular a geometric center of the deployment instrument. It was found that the geometric center of the heart valve prosthesis moves in an aorto-left ventricular direction away from the heart with respect to the deployment instrument/the anatomic structure. In particular at least one label, e.g. (a) the superior or inferior edge of the heart valve prosthesis, (b) the change of a first radial dimension at the distal location in relation to the change of the second radial dimension at the proximal location, (c) the varying open cell size of the heart valve prosthesis, and/or (d) the geometric center of the balloon of the balloon catheter, as a valve prosthesis assembly characteristic may be provided/generated in/based on the baseline image data for training. This can be caused by a predetermined deployment of the heart valve prosthesis in an asymmetric manner, the interaction with the hemodynamics, and/or interaction with the anatomic structure. Hence, including a correction for this effect in the deployment model may avoid malpositioning of the heart valve prosthesis in particular the aortic heart valve prosthesis, in the native anatomic structure, in particular the aortic heart valve.

The heart valve prosthesis assembly characteristic may comprise a first radial dimension at at least a first longitudinal location of the heart valve prosthesis and a second radial dimension at at least a second longitudinal location of the heart valve prosthesis different than the first longitudinal location.

The heart valve prosthesis assembly characteristic may comprise a shape of the heart valve prosthesis in the anatomic structure. The deployment model may be configured for determining deviations of this shape with respect to a predetermined shape of the heart valve prosthesis without interactions with the anatomic structure.

The deployment model may be trained for detecting a non-mirror symmetric deployment of the heart valve prosthesis, in particular in two opposing lateral direction from a center of the heart valve prosthesis. The deployment model may be configured for detecting if one lateral side of the heart valve prosthesis deploys to a different degree than an opposing lateral side. The deployment model may be configured to detect two opposing lateral dimensions which extend from a geometric centerline of the delivery instrument laterally away. By detecting the relative change between these opposing lateral dimensions, the non-mirror symmetric deployment may be inferred. The deployment model may be configured for predicting the future deployment state by including for the non-mirror symmetric deployment.

The point-to-point or line-to-line distance may be a distance between the heart valve prosthesis and a medical instrument which may comprise a catheter and/or a guidewire having a predefined shape.

The anatomic characteristic may be selected from (i) an aortic annulus radial dimension, an aortic root radial dimension, a sinotubular junction radial dimension, and/or a left ventricular outflow tract radial dimension, (ii) a two- or three-dimensional anatomic shape of the native anatomic structure, in particular including changes to the anatomic shape due to a respiratory or cardiac rhythm, (iii) a blood pressure measurement, (iv) a blood flow measurement, (v) a respiratory and/or cardiac rhythm measurement, and/or (vi) alterations of the anatomic structure due to an external factor, in particular an application of rapid pacing, in particular a frequency and/or duration of the rapid pacing application.

Selecting the anatomic characteristic of at least one of (i) - (ii) enables to take the anatomy of the patient into account. Accounting for size and shape of the anatomy enables personalized surgical planning, enhancing procedural accuracy and safety by tailoring interventions to individual patient anatomies. This may further be enhanced by considering the dynamics of the patient's anatomy such as the blood pressure or blood flow (see (iii) and (iv)) or the respiratory and/or cardiac rhythm (see (v)). Selecting the anatomic characteristic of (vi) enables to extract the temporal deformations of the anatomic structure and/or infer a reduction of blood flow due to the external factors, such as a rapid pacing frequency, based on the baseline image data. The anatomic characteristic may include the alterations of (iv) and the two- or three-dimensional anatomic shape of (i), to further enhance the predictive capabilities of the behavior of the medical instrument.

The alterations of the anatomic structure may be used to determine the rapid pacing frequency and/or duration directly based on the baseline image data. Alternatively, to extracting the frequency and/or the duration of rapid pacing based on the intra-operative image sequence, this data may be directly provided for training the deployment model. However, it is beneficial to extract this data only based on the baseline image sequence such that the deployment model can be trained only based on the baseline image sequence without necessitating further data input.

Applying rapid pacing typically includes inducing a heartbeat around 160 - 180 beats per minute (bpm) to facilitate valve prosthesis position/deployment. While rapid pacing is applied, the heart valve prosthesis can be safely deployed while minimizing the risk of dislodgment or improper positioning. If rapid pacing is applied the blood flow is reduced while the native heart valve contracts rapidly as long as rapid pacing is applied. Rapid pacing may for example be applied to the heart via a temporary pacing catheter, in particular a transvenous temporary pacing catheter.

The baseline image sequence and/or the intra-operative image sequence may comprise images showing application of rapid pacing to the heart. Alternative, the baseline image sequence and/or the intra-operative image sequence may be devoid of any images showing application of rapid pacing to the heart. Thereby, the deployment model may be trained/computationally modelled to predict the intra-operative future deployment state without necessitating complex additional medical instruments for delivering rapid pacing to the heart.

The heart valve prosthesis assembly characteristic, optionally anatomic characteristic, may be monitored for different deployment stages included in the baseline image data. This may include (i) monitoring a position and/or an orientation of the heart valve prosthesis with respect to a deployment instrument, in particular a balloon catheter. In alternative or addition, this may include (ii) monitoring a position and/or an orientation of a medical navigation and/or deployment instrument functionally delivering and/or deploying the heart valve prosthesis with respect to the anatomic characteristic. The anatomic characteristic may comprise or consist of the two- or three-dimensional anatomic shape, the aortic annulus radial dimension, the aortic root radial dimension, the sinotubular junction radial dimension, and/or the left ventricular outflow tract. In alternative or addition, this may include (iii) adjusting the heart valve prosthesis assembly characteristic, and optionally the anatomic characteristic, to correct for an obstruction of view and/or a parallax phenomenon, if any is detected during the different deployment stages.

Step (i) may enable to optimize the predictive capabilities of the deployment model dependent on the different deployment stages by identifying movement patterns of the heart valve prosthesis with respect to the deployment instrument, e.g. by identifying slippage/or non-uniform deployment of the heart valve with respect to the balloon catheter during deployment.

By considering the position/orientation with respect to the anatomic characteristic during different deployment stages according to (ii), the change in position/orientation can be correlated with respect to the anatomic characteristic.

The heart valve prosthesis assembly/anatomic characteristic may be influenced during the different deployment stages by different factors at a varying degree, e.g. the varying hemodynamics or non-linear valve migration/slippage. Monitoring the position/orientation in this manner during different deployment stages, preferably throughout all deployment stages, enables to identify which heart valve prosthesis assembly characteristic and/or anatomic characteristic influences the different deployment stage to which degree.

Adjusting the heart valve prosthesis assembly characteristic to account for the obstruction of view and/or the parallax phenomenon according to (iii) may provide an automated error correction ensuring a more reliable and precise deployment model of the medical instrument. Thereby, an inaccurate determination of the position/orientation of the heart valve prosthesis with respect to the anatomy of the patient can be avoided or corrected for.

The deployment model may be configured for predicting the intra-operative future deployment state taking into account (i) a relationship between the change in length of the heart valve prosthesis with a radial expansion of the heart valve prosthesis. The deployment model may be configured for predicting the intra-operative future deployment state taking into account (ii) a relationship between the type of the heart valve prosthesis and a predicted radial expansion and/or change in length of the heart valve prosthesis. In alternative or addition, the deployment model may be configured for predicting the intra-operative future deployment state taking into account (iii) a movement of a geometric center of the heart valve prosthesis and/or a change in orientation of the heart valve prosthesis. In alternative or addition, the deployment model may be configured for predicting the intra-operative future deployment state taking into account (iv) a non-uniform expansion pattern of the heart valve prosthesis during the different deployment stages. The non-uniform expansion pattern may be determined based on detecting (I) a change of a first radial dimension at a distal location with respect to a change of a second radial dimension at a proximal location of the heart valve prosthesis or (II) a varying open cell size of the heart valve prosthesis.

Including a relationship according to (i) enables to determine the deployment stage of the heart valve prosthesis in a.reliable manner in a less error-prone manner by having regard to the longitudinal and radial dimension. The relationship according to (ii) enables to take into account the different expansion properties of different types of heart valve prosthesis. Modelling the movement of a geometric center and/or change in orientation of the heart valve prosthesis according to (iii) provides a simple and robust model.

Predicting based on a non-uniform expansion pattern during the different deployment stages according to (iv) enables more accurate modeling of real-life dynamics of heart valve prosthesis under varying operational conditions at different deployment stages. Including the non-uniform expansion pattern via the varying open cell size may enable a detection of an asymmetrical deformation of the varying cell size of the heart valve prosthesis during the deployment.

Determining the dimensions at a distal and proximal location allows to consider non-uniform deployment of the heart valve prosthesis.

The open cells may have different sizes/shapes. The open cells may have different shapes/sizes at different longitudinal sections of the heart valve prosthesis. The cell sizes/shapes of a stent of the heart valve prosthesis at different longitudinal sections deform non-uniformly during deployment. Different longitudinal sections may contract longitudinally to a different degree when being deployed and correspondingly also expand radially to a different degree. This may lead to a displacement of the heart valve prosthesis with respect to a delivery device.

The cell sizes/shapes also deform when engaging the anatomic structure such that certain cell sizes/shapes vary to compensate for a contour of the anatomic structure. Also based on this compensation of the varying cell sizes/shapes the non-uniform pattern may be inferred by the deployment model.

This modelling of the non-uniform expansion pattern based on varying cell size may further be used to infer three-dimensional shape information of the heart valve prosthesis based only on two-dimensional image sequences. This may be achieved based on the cell sizes being arranged in a predetermined three-dimensional relationship to each other in a known deployment pattern and design. The baseline image sequence typically provides two-dimensional data, i.e. a two-dimensional projection of the three-dimensional cell shape/size deployment pattern. Based on this two-dimensional projection, the three-dimensional shape information, and thus the three-dimensional non-uniform expansion pattern, of the heart valve prosthesis may be reconstructed.

A deployed shape of the heart valve prosthesis or a deployment pattern of the heart valve prosthesis without any additional forces exerted on the heart valve prosthesis, i.e. not subject to confinement of an anatomic structure, may be provided for training the deployment model such that the non-uniform expansion pattern may be detected more reliably.

The deployment model may be a biomechanical model which includes the two- or three-dimensional anatomic shape and is configured to simulate and/or evaluate the deployment of the heart valve prosthesis. The deployment model may be selected from at least one of (i) a quasi-static deployment model, (ii) a two-dimensional finite element model, optionally comprising a plurality of beam elements, (iii) a three-dimensional finite element model, (iv) a dynamic finite element model, and (v) a computational fluid dynamics model.

This allows to take biomechanical interactions into account to generate the deployment model, in particular in real-time.

The dynamic three-dimensional finite element model may be adapted for determining forces between the heart valve prosthesis and the anatomic shape during deployment.

The computational fluid dynamics model may be adapted for predicting valve regurgitation for the deployed heart valve prosthesis.

The method may comprise of generating a plurality of deployment models based on the baseline image data. The baseline image data, and in particular additional image data, received, by the one or more processors, may comprise or consist of a statistical atlas of different patients comprising the respective anatomic structure of the different patients.

The geometry of the heart valve in the dynamic three-dimensional model may be modelled either by beam, shell, or solid elements. The quasi-static model and/or dynamic three-dimensional model may include a plurality of positions, orientations and/or deployment speeds to deploy the heart valve prosthesis.

The anatomic shape may comprise a radial dimension or shape of the aortic valve annulus, a radial dimension or shape of the left ventricular outflow tract, and/or a radial dimension or shape of the aortic root. The deployment model may be configured for modelling the position, the shape, and/or a force on the anatomic shape or a fully deployed heart valve prosthesis having a cylindrical, conical, or funnel shape within the anatomic shape.

This enables to provide a deployment model which is adapted for accounting for a deployment behavior of the heart valve prosthesis in these regions of interest of the native anatomy. In addition, the deployment model may include the type/size of the heart valve prosthesis.

The deployment model can thereby include the relevant boundary conditions for deploying the valve prostheses which has a cylindrical, conical, or funnel shape.

A plurality of deployment models may be determined independently for subgroups of the baseline image data, subgroups of the heart valve prosthesis assembly characteristic, and/or sub-groups of the anatomic characteristic.

The subgroups of the baseline image data, the subgroups of the heart valve prosthesis assembly characteristic and/or the subgroups of the anatomic characteristic may be determined based on a clustering analysis. In addition or alternative, PCA may be used for dimensionality reduction. This may enable for classifying the data according to the underlying similarities within data in the subgroup in a reliable manner rather than categorizing according to specific individual features, e.g. the type of heart valve prosthesis. Moreover, this enables a reduced complexity of training the computer-implemented model without necessitating complex supervised learning algorithms.

Using this plurality of different deployment models allows to optimize the prediction capabilities for the deployment of the heart valve prosthesis assembly by excluding spurious correlation which merely apply to a subgroup of certain types of heart valve prostheses, radial/longitudinal dimensions of the types, and/or different anatomic structures. Thereby, a more versatile plurality of deployment models or an adaptive generative model combining the plurality of deployment models may be generated which is fine-tuned for a variety of the previously mentioned subgroups.

The plurality of deployment models may be determined for each different type of heart valve prosthesis, each different radial/longitudinal dimension of the type of heart valve prosthesis, and/or each different anatomic structure of different patients individually.

The baseline image sequence may comprise baseline image sequence of a plurality of different medical instruments, in particular different heart valve prosthesis, a plurality of different patients, and in particular a plurality of different anatomic characteristics. The deployment model may be generated for each of the above-mentioned independently and the adaptive generative model may be generated based on the plurality of the deployment models.

The adaptive generative model may be used instead of the deployment model to predict the intra-operative future deployment state of the heart valve prosthesis based on the intra-operative image sequence comprising the heart valve prosthesis assembly in the current deployment state. This may increase versatility and effectiveness of the trained model by the model being specifically optimized for a plurality of different subgroups individually..

Alternatively, the adaptive generative model may be generated based only on a plurality of different heart valve prosthesis assembly characteristics, and optionally the anatomic characteristic, directly without generating the at least one deployment model beforehand.

The adaptive generative model may be adapted for predicting the deployment of the heart valve prosthesis assembly based on the deployment model of the previously described plurality of deployment models and based on a threshold similarity of the baseline image sequence, the heart valve prosthesis assembly, and/or the anatomic characteristic with a corresponding intra-operative image sequence, an intra-operative heart valve prosthesis assembly characteristic, and/or an intra-operative anatomic characteristic.

This enables the adaptive generative model to apply the deployment model having the most similarities to the intra-operative data.

The plurality of deployment models may be determined for types of heart valve prosthesis, different radial/longitudinal dimensions of the type of heart valve prosthesis and/or anatomic structures of the patient. The deployment model of the plurality of deployment models may be selected based on the threshold similarity with the heart valve prosthesis assembly, in particular the heart valve prosthesis or the deployment/navigation instrument. In addition or alternative, the deployment model may be selected based on the intra-operative anatomic structure or a threshold similarity with the intra-operative anatomic structure visible within the intra-operative image sequence.

Alternatively, the adaptive generative model may be adapted to receive a user input indicative of the type of heart valve prosthesis, the different radial/longitudinal dimension of the type of heart valve prosthesis, and/or the anatomic structure of patients to select and apply a corresponding deployment model.

Another aspect of the invention relates to a computer program comprising instructions which, when the program is executed by a computer, cause the computer to carry out the previously described method.

Another aspect of the invention relates to a non-transitory computer-readable medium having stored there on a computer program product comprising code which, when executed on a processor carries out the previously described computer-implemented method.

The method may comprise training the machine-learning model to determine and output at least one control command for deploying and/or moving the heart valve prosthesis to achieve the predicted intra-operative future deployment state. Alternatively or additionally, the machine learning model may be trained to determine display data based on the predicted deployment for displaying instructions for achieving the intra-operative future deployment state on a display in real-time. The control command / display data may be configured to achieve the intra-operative future deployment state without applying rapid pacing to the heart.

Another aspect of the invention relates to a trained machine-learning model for predicting an intra-operative future deployment state of a heart valve prosthesis assembly based on an intra-operative image sequence comprising the heart valve prosthesis assembly trained according to the previously described computer implemented method.

Another aspect of the invention relates to a computer-implemented method of predicting a deployment of a heart valve prosthesis assembly which comprises receiving, by one or more processors, an intra-operative image sequence. The intra-operative image sequence comprises images which include the heart valve prosthesis assembly in a current deployment state. The method comprises analysing the intra-operative image sequence, at least one image of the intra-operative image sequence including the heart valve assembly characteristic of the heart valve prosthesis assembly. The method comprises predicting an intra-operative future deployment state of the heart valve prosthesis assembly using a deployment model, in particular a deployment model trained using a previously described computer implemented method, based on the intra-operative image sequence.

The deployment model may be configured for compensating the predicted intra-operative future deployment state taking into account at least one of the previously described relationships, movement of the geometric center, and/or non-uniform expansion pattern.

The intra-operative future deployment state may include a virtual future geometric center of the heart valve prosthesis assembly which is displaced relative to a deployment instrument in an axial direction of the heart valve prosthesis assembly with respect to a current geometric center of the current deployment state. The virtual future geometric center of the heart valve prosthesis may be displayed in a proximal axial direction with respect to the current geometric center of the current deployment state.

The terms "proximal" and "distal" are defined from the perspective of a clinician advancing the heart valve prosthesis assembly distally towards the heart and retracting the heart valve prosthesis proximally away from the heart.

The method may include determining the output data. The output data may comprise display data for displaying the intra-operative future deployment state on a display. The output data may comprise display data for displaying an initial intra-operative deployment state to achieve the intra-operative future deployment state through deployment from the initial intra-operative deployment state.

The output data may comprise display data for displaying user instructions to achieve the intra-operative future deployment state on the display. The output data may include risk data indicative of a risk assessment of the intra-operative future deployment state, in particular with respect to the anatomic structure. The output data may include deployment parameters for achieving the intra-operative future deployment state.

The display data may include a model of the heart valve prosthesis assembly to provide a more realistic and tangible representation for clinicians.

Another aspect of the invention relates to a medical apparatus for positioning and deploying a heart valve prosthesis assembly.

The medical apparatus comprises an input interface for receiving an intra-operative image sequence, in particular an intra-operative fluoroscopy image sequence, preferably a pre-operative computer tomography image sequence. The medical apparatus comprises a processing unit which includes an interface for accessing the previously described deployment model or comprises the previously described model, in particular the previously described model generated by the previously described computer-implemented learning method. The processing unit is adapted for predicting an intra-operative future deployment state of the intra-operative heart valve prosthesis assembly based on the deployment model and the intra-operative image sequence comprising the heart valve prosthesis assembly in a current deployment state. The processing unit is further adapted for generating output data comprising at least one control instruction for deploying and/or moving the heart valve prosthesis assembly based on the predicted intra-operative future deployment state. The control instruction can be either a control command or display data being displayable on a display for achieving the intra-operative future deployment state in real-time. The medical apparatus comprises an output interface for transmitting the at least one output data to a robotic controller adapted for carrying out the control command and/or to the display.

This enables to reliably move/deploy the heart valve prosthesis assembly based on the deployment prediction of the heart valve prosthesis assembly intra-operatively of the deployment model. The medical apparatus is preferably adapted for generating the output data in real-time.

The processing unit may be adapted for predicting the deployment of the intra-operative heart valve prosthesis assembly during different deployment stages based on the pre-operative computer tomography image sequence of the patient in addition to the intra-operative image sequence of the patient.

The processing unit may be adapted for generating at least one control instruction for deploying and/or moving the heart valve prosthesis assembly based on the predicted intra-operative future deployment state without applying rapid pacing to the heart.

To position/deploy the heart valve prosthesis according to the intra-operative final deployment state without applying rapid pacing to the heart, necessitates a much faster positioning of the heart valve prosthesis assembly. The resting heart rate is typically ranged from 40 - 60 bpm for athletes and 60 - 100 bpm for average adults. The target position such as a heart valve prosthesis assembly typically assumes a completely closed position every heartbeat such that interaction of an heart valve prosthesis in the predicted intra-operative final deployment state may lead to dislodgement of plaque.

In order to achieve a positioning of the heart valve prosthesis without rapid pacing, the control command may be configured to operate a robotic controller to advance the heart valve prosthesis between two resting heart beats.

The control command may be configured for operating a robotic controller to axially move the heart valve prosthesis.

The control command may be configured to axially move the heart valve prosthesis via a robotic controller with a velocity of at least 15 mm/s, in particular at least 25 mm/s, preferably at least 35 mm/s.

This velocity in an axial direction to position the heart valve prosthesis may enable the crossing of the native heart valve while reducing the risk of plaque risk of plaque dislodgement.

The control command may be configured for operating a robotic controller for deploying the heart valve prosthesis from a non-deployed state to a fully deployed state in less than 1 s, in particular in less than 0.7 s, preferably, in less than 0.5 s.

These deployment velocities, e.g. by inflating a balloon of a balloon catheter on which the heart valve prosthesis is mounted, enable a deployment without necessitating rapid pacing.

The processing unit may be adapted for tracking a heart valve prosthesis of the heart valve prosthesis assembly, in particular a terminal proximal end of the heart valve prosthesis of the heart valve prosthesis assembly and/or a terminal distal end of the heart valve prosthesis based on detecting a radiopaque section of the heart valve prosthesis in the intra-operative image sequence.

This simplifies a detection of the heart valve prosthesis and provides a more reliable localization within the intra-operative image sequence.

The processing unit may be adapted for tracking the current deployment configuration of a balloon catheter, e.g. by detecting a radiopaque fluid for inflating the balloon catheter during deployment of the heart valve prosthesis in the intra-operative image sequence.

The processing unit may be configured for tracking a deployment instrument and/or a navigation instrument of the heart valve prosthesis assembly, in particular a center location of the deployment instrument, based on detecting at least one radiopaque section of the deployment instrument and/or the navigation instrument.

The processing unit may be configured to determine a distance between the tracked position of the deployment/navigation instrument, in particular the center location of the deployment instrument, and the terminal proximal and/or the terminal distal end of the heart valve prosthesis.

The processing unit may be adapted to determine the displacement of the geometric center of the heart valve prosthesis in the intra-operative future deployment state with respect to the current deployment state by comparing the distance between the tracked positions, preferably the distance between the center location of the deployment instrument with respect to the terminal proximal end and/or the terminal distal end of the heart valve prosthesis. Thereby, the non-uniform expansion pattern may be inferred and included in the deployment model.

The processing unit may be adapted for determining at least one inflation control command for a partial inflation of the balloon catheter for deploying the heart valve prosthesis to an intermediate deployment configuration, in particular such that a balloon of the balloon catheter is inflated between 50% and 90%, preferably between 65% and 85%, in particular between 70% and 80%, with respect to a final deployed configuration of the balloon of the balloon catheter. The processing unit may be adapted for thereafter determining at least one intermediate positioning control command for repositioning of the heart valve prosthesis with respect to the anatomic structure.

The final deployed configuration of the balloon is defined as essentially conforming to a radial dimension equal or larger than a radial dimension of the native anatomic structure in which the heart valve prosthesis is deployed.

This allows for adjusting the position/orientation of the heart valve prosthesis in accordance with the predicted final deployed configuration based on the intermediate deployment configuration to allow more accurate placement. The change in position/orientation of the heart valve prosthesis from the intermediate deployment configuration to the final deployment configuration is strongly influenced by the interaction with the cardiac vasculature, a radial shift of the heart valve into a centric position of the anatomic structure, and the hemodynamics. The control command may be more precisely predicted based on the intermediate deployment configuration instead of the non-deployed configuration.

The medical apparatus may comprise a robotic controller for operating and moving the medical instrument for deployment of the heart valve prosthesis.

This medical apparatus may enable a partially or fully automated control of the heart valve prosthesis deployment and optimized real-time interaction between software and hardware to enable more precise positioning/orienting of the heart valve prosthesis.

The at least one control command may be configured for operating the robotic controller to deploy and/or move the heart valve prosthesis, in particular to move the heart valve prosthesis in a longitudinal direction, a lateral direction, and/or to tilt the heart valve prosthesis or the medical deployment/navigation instrument.

This enables deployment and/or positioning/orienting of the heart valve prosthesis in different degrees of freedom to enable a more precise placement at a target implantation depth. The robotic controller may be adapted to bend at least one subsection of the delivery system proximal the balloon catheter/heart valve prosthesis, e.g. positioned in the aortic arch/aortic root, to achieve a movement in the lateral direction and/or the tilt of the heart valve prosthesis.

The robotic controller may achieve a movement of the heart valve prosthesis in a lateral direction by operating the balloon inflation such that the heart valve prosthesis can be concentrically arranged in the anatomic structure.

The processing unit may be adapted for predicting the behavior of the intra-operative heart valve prosthesis assembly based on sensor data, in particular sensor data receivable from the robotic controller.

The processing unit may be configured for applying a sensor fusion algorithm, in particular a Kalman Filter algorithm, to the sensor data and in particular data generated by the deployment model to generate the control command.

Additionally or alternatively, the processing unit may be adapted for applying the sensor fusion algorithm to the intra-operative medical image sequence, the intra-operative heart valve prosthesis assembly characteristic, and/or the intra-operative anatomic characteristic to generate the control command.

This may provide a multivariate feedback loop between the processing unit and the robotic controller. The sensor data may include encoder data of at least one actuator of the robotic controller. The sensor data may include sensor data indicative of a pressure/force exerted on the delivery system and/or on the heart valve prosthesis.

The processing unit may be adapted to include a simple mechanical model of the delivery system in combination with the Kalman Filter algorithm to further optimize a position/orientation accuracy.

Another aspect of the invention relates to a method for positioning and deploying a heart valve prosthesis assembly. The method comprises receiving an intra-operative image sequence, in particular an intra-operative fluoroscopy image sequence, and preferably pre-operative computer tomography data. The method includes predicting an intra-operative future deployment state of the heart valve prosthesis assembly comprising a heart valve prosthesis during different deployment stages of the heart valve prosthesis based on the intra-operative image sequence and a deployment model, in particular generated by the computer-implemented method as previously described. The method comprises generating output data including at least one control instruction for deploying and/or moving the heart valve prosthesis based on the predicted intra-operative future deployment state. The control instructions are either a control command or display data comprising user instructions for displaying instructions on a display for achieving the intra-operative future deployment state in real time. The method further includes transmitting the at least one output data to the display and/or a robotic controller adapted for positioning and deploying the heart valve prosthesis to the intra-operative future deployment state. The method further comprises deploying and/or positioning the heart valve prosthesis, in particular moving the heart valve prosthesis in a longitudinal direction and/or tilting the heart valve prosthesis or the medical deployment/navigation instrument in a partially or fully automated manner by the robotic controller or manually based on instruction on the display.

Further embodiments of the invention and improvement of the described embodiments will become apparent with the following description of the embodiments.

The invention is now described with reference to certain embodiments and figures which show:
Figures 1A - 1B: a schematic cross-sectional view of a heart with a medical apparatus which was advanced through the aortic root to the native aortic valve deploying a heart valve prosthesis,
Figure 1C: a cross-sectional view of the heart with a fully deployed heart valve prosthesis within the native aortic valve,
Figure 1D: a cross-sectional view of the aortic root, the native aortic heart valve, and a subsection of the left ventricle,
Figures 2A - 2C: side views of a heart valve prosthesis moving from a non-deployed configuration to an intermediate deployment configuration to a fully deployed configuration,
Figures 2D - 2F: a cross-sectional view of the heart valve prosthesis showing different deployment behaviours,
Figures 2G and 2H: a cross-sectional view of anatomic structures to which a heart valve prosthesis in a fully deployed configuration comprising a differently shaped proximal section and a differently tapered distal section may conform,
Figure 3: a schematic illustration of the medical apparatus comprising a robotic controller for moving and deploying the heart valve prosthesis based on an image sequence intra-operatively, and
Figure 4A: a graph showing a mean, a median, and a standard deviation of a length of the heart valve prosthesis changing throughout the images acquired during the deployment within 147 patients,
Figure 4B: a subsection of the graph of Fig. 4A corresponding only to the deployment process of the heart valve prosthesis,
Figures 5A and 5B: a fluoroscopy image showing the superior edge and an inferior edge of the heart valve prosthesis and a fluoroscopy image showing a balloon superior and a balloon inferior edge markers and a balloon center marker,
Figure 6A: a mean, a median, and a standard deviation of the distance from a superior edge of the heart valve prosthesis to a balloon center marker for the 147 patients,
Figure 6B: a mean, a median, and a standard deviation of a distance from an inferior edge of the heart valve prosthesis to a balloon center marker for the 147 patients,
Figure 6C: a mean, a median, and a standard deviation of the heart valve prosthesis centroid shift of Figs. 6A and 6B, and
Figures 6D and 6E: a first fluoroscopy image of the heart valve prosthesis assembly in a non-deployed state and a second fluoroscopy image of the heart valve prosthesis in a fully deployed state,
Figures 7A and 7B: a side view of a heart valve prosthesis in a non-deployed configuration and a fully deployed configuration comprising varying open cell sizes,
Figures 7C and 7D: a perspective side view of the heart valve prosthesis of Figs. 7A and 7B in the non-deployed configuration and the fully deployed configuration,
Figure 8: a graph of an average circumferential width of open cells of a stent of the heart valve prosthesis in the non-deployed configuration and the fully deployed configuration according to Figs. 7A to 7D.

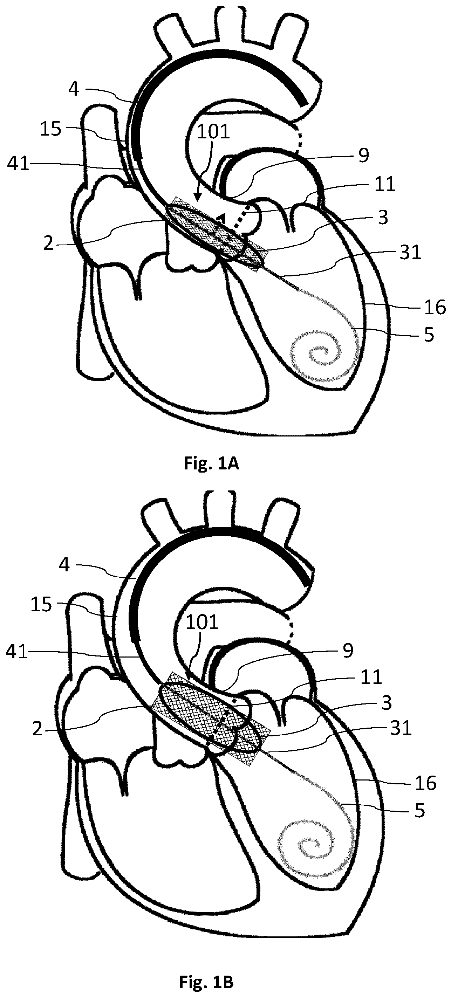

Figures 1A and 1B show a schematic cross-sectional view of a heart with a medical apparatus 101 which was advanced through the aortic root 15 for deploying a heart valve prosthesis 2.

Figure 1A shows the heart valve prosthesis 2 of a heart valve prosthesis assembly in a non-deployed configuration partially arranged within the native aortic annulus 11. The heart valve prosthesis assembly further comprising a navigation instrument 4 formed by a steerable catheter was used for advancing the heart valve prosthesis 2 transfemorally to the aortic valve. A distal section of the heart valve prosthesis assembly comprises a more flexible section 41 which is enclosed by an outer sheath of the navigation instrument 4 and can be extended and retracted from the outer sheath. This flexible section 41 is more controllable and facilitates positioning/orienting of the heart valve prosthesis 2 with respect to the native anatomic structure formed by the aortic annulus 11. Distally from the flexible section 41, the delivery system comprises a deployment instrument 3 which includes a balloon catheter 31. The heart valve prosthesis 2 is mounted on an outer surface of a balloon of the balloon catheter 31 such that it is radially expandable to be deployed by fluid inflation of the balloon. A medical instrument formed by a coiled ventricular positioning guidewire 5 which has a coiled shape is partially supported in proximity of the ventricular apex of the left ventricle 16 and forms the distal most section of the delivery system. The positioning guidewire 5 enables to advance the heart valve prosthesis 2 through the aortic annulus 11 in a controlled manner. The delivery system is advanced through the aortic annulus 11 while rapid pacing is delivered such that the heart valve leaflets enable passage through the aortic valve.

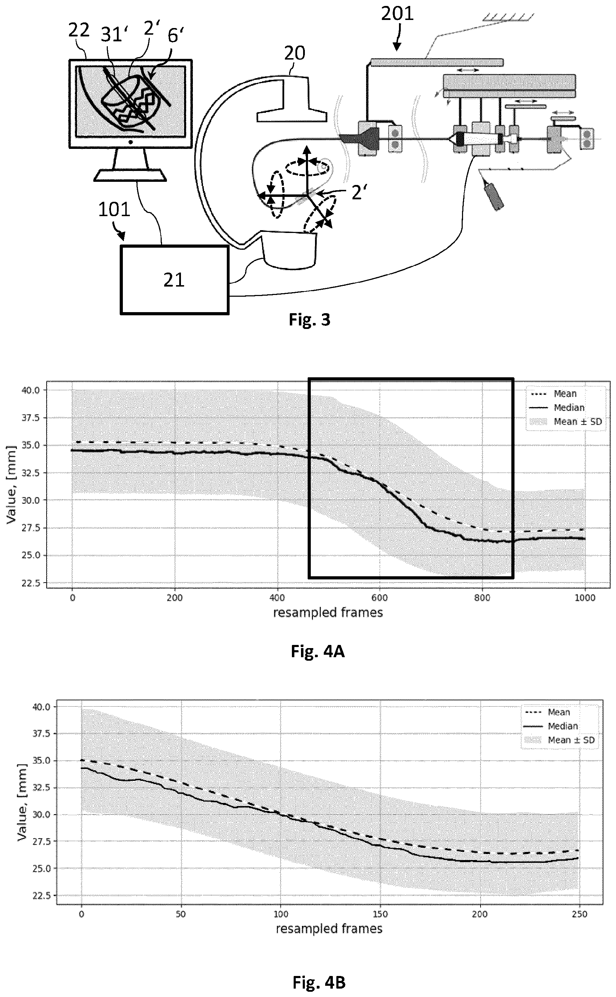

A medical apparatus for operating the delivery system is adapted for providing control commands for a robotic controller (see Fig. 3) to longitudinally position the delivery system based on an image sequence. Moreover, the flexible end 41 of the delivery system is tiltable away from a curvilinear axis of the delivery system and rotatable with respect to the native anatomic structure based on control commands of the medical apparatus. The image sequence supplied to achieve this position/orientation of the heart valve prosthesis 2 is a fluoroscopy image sequence which provides two-dimensional information of the heart valve prosthesis 2 with respect to the anatomy during an administration of contrast agent.

Figure 1B shows the heart valve prosthesis 2 in an intermediate deployment configuration within the aortic annulus 11. This intermediate deployment configuration is achieved by partially inflating the balloon of the balloon catheter 31 by 50% - 90% of the maximum fluid inflation volume with respect to a final deployed configuration. As shown in Fig. 1B as compared to Fig. 1A, the heart valve prosthesis 2 moves during the deployment process, especially in a lateral direction (see Fig. 1A, dashed arrow indicating the direction of movement). The balloon catheter 31 constricts the blood flow to a varying degree during deployment and thereby influences the hemodynamics. A position/orientation of the heart valve prosthesis 2 is further influenced by the mechanical properties of the delivery system, a respiratory and cardiac rhythm, and a frequency of rapid pacing. A displacement during deployment via the balloon catheter 31 can further be caused by inherent non-uniform dynamics of the balloon and eccentric positioning of the heart valve prosthesis (see Fig. 1A), e.g. due to conforming to a curvature of the aortic arch. The heart valve prosthesis type and/or size and interaction of the medical instruments with the vasculature may further lead to displacement. All these effects can contribute to displacing the heart valve prosthesis 2 with respect to the native anatomic structure.

The medical apparatus is further adapted for compensating for a non-linear expansion pattern of the heart valve prosthesis 2 during the expansion via the balloon. The non-linear expansion pattern can be inherent by different cell sizes and/or shapes of the heart valve prosthesis 2. During expansion of the heart valve prosthesis 2 the different cell sizes and/or shapes lead to a non-uniform expansion, i.e. longitudinal subsections of the heart valve prosthesis 2 change in length differently during a radial expansion of the heart valve prosthesis 2. The model is adapted to predict this non-uniform expansion pattern based on including the varying open cell sizes/shapes to determine an intra-operative final deployment state in a reliable manner. The balloon expands the heart valve prosthesis with a uniform pressure, so that this nonlinearity can further lead to slippage and, in interaction with this nonlinear expansion, can displace the heart valve prosthesis.

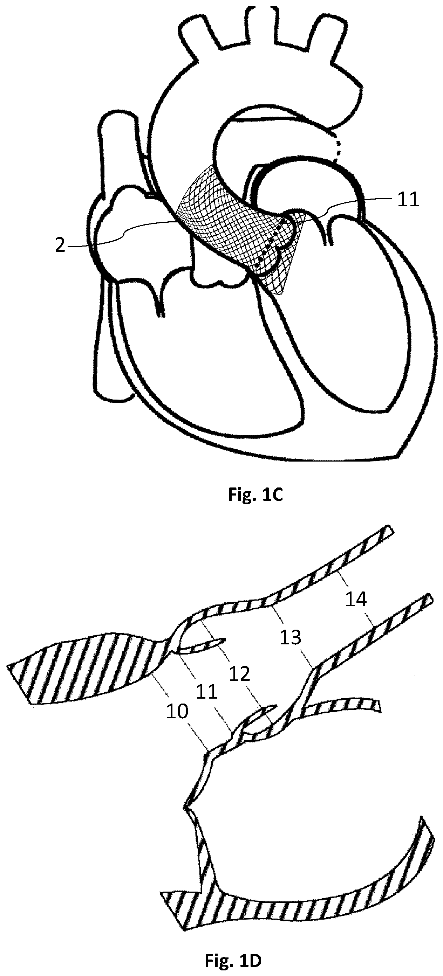

The positioning/orienting of the heart valve prosthesis 2 is typically dependent on the expertise of clinicians predicting the movement during deployment such that the heart valve prosthesis 2 can be positioned to manually counteract the plurality of different effects. However, since the prediction is contingent on a multifactorial array of determinants, the behaviour of the heart valve prosthesis 2 is difficult to predict. The prediction is influenced by a clinician's learning curve, manual imprecision, cognitive overload, and involuntary subjective misjudgement. Hence, the final deployed configuration (see Fig. 1C) of the heart valve prosthesis 2 may be suboptimal resulting in potential risk of valve migration, paravalvular leakage, flow obstruction, embolism, obstruction of the coronary arteries (not shown in Figs. 1A - 1C) or even impairing the function of the adjacent mitral valve.

Figure.1C shows a cross-sectional view of the heart with a fully deployed heart valve prosthesis 2. The heart valve prosthesis 2 assumes a final deployment configuration at a target implantation depth. The implantation depth of a heart valve prosthesis 2 is defined as the distance from the inflow edge of the heart valve prosthesis 2 to a reference anatomical landmark, such as the native valve annulus, measured along the longitudinal axis of a delivery catheter of the heart valve prosthesis assembly. As shown in Fig. 1C, the heart valve prosthesis 2 has a predetermined shape to conform the cardiac vasculature with a tapered distal shape to conform to the left ventricular outflow tract. The heart valve prosthesis 2 may have an essentially cylindrical shape in a non-deployed state. The heart valve prosthesis 2 may be changing shape to a non-cylindrical, in particular a conical or a funnel shape, the conical or funnel shape preferably comprising at least one curved surface. The heart valve prosthesis 2 change shape based on the enveloping anatomic structure. Alternatively, the heart valve prosthesis may be a self-expandable heart valve prosthesis, in particular a self-expandable heart valve prosthesis which has a predetermined shape. The target implantation depth is defined to optimize the position of the heart valve prosthesis 2 with respect to an anatomic structure formed by the aortic valve annulus of the patient and the type/size of the heart valve prosthesis 2 and may be provided by a clinician to the medical apparatus for generating the control commands.

Corresponding image sequences acquired during a plurality of these stages shown in Figs. 1A - 1C which are carried out manually are used as a baseline image sequences to train the medical apparatus and carry out predictions of the behavior of the medical instrument intra-operatively. Subsequently, if the medical apparatus is confronted with an intra-operative image sequence, an accurate prediction of the intra-operative behaviour can be generated. A plurality of baseline image sequences comprising the deployment of different types and sizes of heart valve prostheses 2 within different patients enables the medical apparatus to effectively predict the behavior of a plurality of medical instrument(s).

The method includes generating a deployment model for different subgroups to optimize the prediction for different use cases separately, e.g. types of heart valve prosthesis 2, different radial/longitudinal dimension of the type of heart valve prosthesis 2, a frequency of rapid pacing, and/or different types of anatomic structures.

In order to individually analyze the medical baseline image sequence, the image sequence is classified, e.g. by a clustering analysis of the data, preferably followed by a principal component analysis (PCA).

The method further might include determining an adaptive generative model based on the plurality of these deployment models which is adapted for predicting the behavior of different intra-operative medical instruments.

The method includes extracting a heart valve prosthesis assembly characteristic comprising a deployment dynamic of the heart valve prosthesis based on these baseline image sequences. The deployment dynamic comprises a non-linear expansion pattern of the heart valve prosthesis 2. Subsequently, a statistical analysis is carried out to develop an algebraic/statistical model to describe the predicted non-linear expansion pattern of the heart valve prosthesis. To achieve this, radial dimensions of the heart valve prosthesis 2 at different longitudinal positions or the corresponding cell size/shape are monitored to determine a non-uniform expansion pattern of the heart valve prosthesis 2 which is included in the model. Based on this non-uniform expansion pattern, the intra-operative future deployment state of the heart valve prosthesis 2 is generated by the model in a more accurate manner.

The method further includes extracting an anatomic characteristic from the baseline image sequence comprising a two-dimensional anatomic shape of the aortic heart valve. The change of the two-dimensional shape during respiratory and cardiac rhythm might be further included in the model. Another anatomic characteristic included in the model might be a frequency and duration of the heart rate influenced by rapid pacing being applied during the deployment/positioning of the heart valve prosthesis which is derived from the baseline image sequence. In this manner, the model can solely rely on the image sequence to improve the predictive capabilities of the model.

By extracting the heart valve prosthesis assembly characteristic and the anatomic characteristic during the different deployment stages of the heart valve prosthesis 2, the deployment model is optimized for the different stages of deployment which are influenced by multivariate factors at varying degrees. The deployment model is a two-dimensional or three-dimensional and quasi-static or dynamic finite element model which is configured for determining forces between the heart valve prosthesis and the anatomic shape during deployment. Alternatively, the deployment model may be a statistical model or a machine learning model.

Figure 1D shows a cross-sectional view of the aortic root, the native aortic heart valve, and a subsection of the left ventricle. At least one anatomic characteristic used for training the model includes at least one of a two-dimensional anatomic shape comprising a radial dimension of the left ventricular outflow tract 10, the aortic annulus 11, the sinus of vasalva 12, the sinotubular junction 13 and/or a cross-section of the ascending aorta 14. These radial dimensions may be determined based on the fluoroscopy image sequence acquired during the administration of contrast agent. By including different regions of the anatomic structure, in particular the entire two-dimensional anatomic shape, the predictive capabilities of the adaptive generative model for intra-operative instruments may be improved.

Alternatively, the medical apparatus might be adapted to deploy the heart valve prosthesis without using the administration of contrast agent, i.e. no fluoroscopic images indicative of a two-dimensional anatomic shape. Instead, the image sequence(s) to train the model can comprise no images acquired during the administration of contrast agent. In this manner, the model can be trained to predict the intra-operative future deployment state based on the position of the heart valve prosthesis assembly and anatomic structures which are visible without contrast agent. This may facilitate the surgical procedure and improve patient care by omitting contrast agent administration intra-operatively to predict the intra-operative future deployment state.

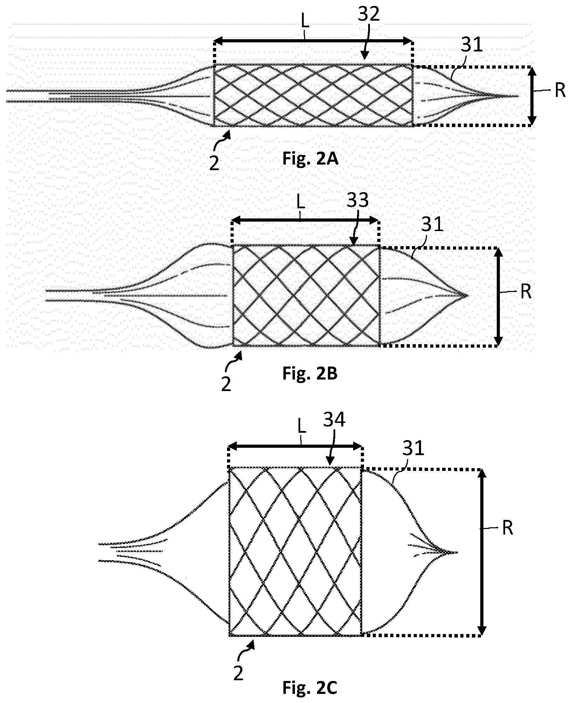

Figure 2A to 2C show side views of a heart valve prosthesis 2 moving from a non-deployed configuration 32 to an intermediate deployment configuration 33, to a fully deployed configuration 34. The heart valve prosthesis 2 is mounted on a balloon of a balloon catheter 31 which is inflated to deploy the heart valve prosthesis 2.

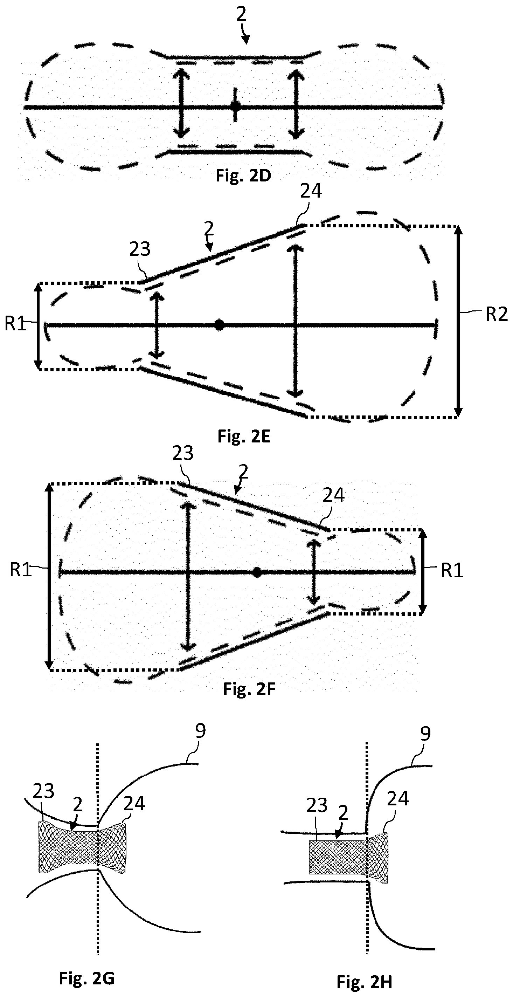

Figure 2A of the heart valve prosthesis 2 in the non-deployed configuration shows a longitudinal dimension L and a radial dimension R of the heart valve prosthesis 2. The ratio between the longitudinal dimension L and the radial dimension R in the non-deployed configuration is large and decreases during the deployment shown in Figs. 2B and 2C. A plurality of these ratios can be determined at different longitudinal sections of the heart valve prosthesis 2 to correct for a non-linear deformation of the heart valve prosthesis 2 and/or the balloon to predict the non-uniform expansion pattern (not shown in Figs. 2A - 2C). A simple example of a non-uniform expansion pattern is described in Figs. 2E and 2F. The processing unit of the medical apparatus is adapted to assess these ratios to facilitate estimation of the intra-operative future deployment state of the heart valve prosthesis 2. The intra-operative future deployment state includes the position of the heart valve prosthesis 2 with respect to the balloon catheter 31 and non-uniformly deformed shape of the heart valve prosthesis 2.

The processing unit is alternatively adapted to determine the future deployment state based only on the expansion of a deployment instrument 3, e.g. an expansion of a balloon of a balloon catheter. The balloon expansion may show the same non-uniform expansion pattern since different sections of the heart valve prosthesis 2 are deformed to a varying degree during the expansion. The balloon of the balloon catheter 31 may be expanded during deployment via radiopaque fluid to be monitored by fluoroscopy imaging.

Additionally or alternatively, the effects of varying open cell sizes of an expansion pattern of a stent of the heart valve prosthesis 2 during different deployment stages may be included in the model to determine the future deployment state.

In a preferred embodiment, the heart valve prosthesis 2 is a balloon-expandable heart valve prosthesis 2 shown in Figs. 2A -2C. Alternatively, the heart valve prosthesis 2 may be self-expandable heart valve prosthesis 2 which is deployed by retracting an outer sheath of the heart valve prosthesis assembly. In this case the non-linear deployment pattern may be predicted by including a sequential increase in cell size originating from a distal end once an outer sheath is retracted for deployment of the self-expandable heart valve prosthesis 2.

Depending on the specific heart valve prosthesis type and/or size, these expansion patterns can be used to pre-calculate and predict the future deployment state of the heart valve prosthesis 2, enabling prior optimal positioning/orienting of the heart valve prosthesis 2 via a robotic controller in advance based on control commands of the medical apparatus. Alternatively, user instructions on how to achieve the intra-operative future deployment state may be generated by the model and displayed on a display.

Figure 2B shows the heart valve prosthesis 2 in the previously described intermediate deployment configuration 33 and Fig. 2C shows the heart valve prosthesis 2 in the fully deployed configuration 34. The processing unit is specifically optimized to predict the deployment behavior of the heart valve prosthesis 2 based on the intermediate deployment configuration to predict the final deployed configuration 34. This provides a more exact prediction of the final deployment position/orientation while the heart valve prosthesis 2 can still be repositioned based on the control command. The influence of the hemodynamics and the interaction with the anatomic structure increases/changes during the movement from the intermediate deployment configuration 33 to the final deployment configuration 34. The method and medical apparatus may be adapted for including a plurality of intermediate deployment configurations, e.g. a first intermediate deployment configuration with a balloon inflation of 10 - 40%, in particular 15 - 30% for a coarse adjustment of the position/orientation of the heart valve prosthesis 2 and a second intermediate deployment configuration with a balloon inflation of 50 - 90%, in particular between 70 - 80% with respect to a final deployed configuration.

The medical apparatus is adapted for positioning the heart valve prosthesis 2 in the intermediate deployment configuration, e.g. by transmitting a control command based on the prediction to a robotic controller or by displaying data comprising instructions for carrying out the control command manually for a clinician which are displayed on a display. Subsequently, the final deployment from an intermediate deployment to the final deployment configuration is carried out in a single rapid step to avoid prolonged increased influences during this stage. This may reduce risks of malpositioning of the heart valve prosthesis 2, adverse effects due to prolonged obstruction of blood flow, and/or dislodgement of plaque.

Figures 2D to 2F show a cross-sectional view of a heart valve prosthesis 2 being deployed in a uniform manner, a distal end 24 expanding radially to a greater degree with respect to a proximal end 23, and the proximal end 23 expanding radially to a greater degree with respect to the expansion of the distal end 24.

The medical apparatus is adapted for modelling non-uniform expansion patterns of the heart valve prosthesis 2 dependent on a current stage of deployment, e.g. the heart valve prosthesis 2 not expanding radially outwardly in a uniform manner from its geometric center. The non-uniform deployment pattern may be based on mechanical constraints of the heart valve prosthesis 2, a predetermined shape of the heart valve prosthesis 2 in the deployed configuration, and/or a non-uniform interaction with the anatomic shape, e.g. the anatomic shape limiting radial expansion at a subsection of the heart valve prosthesis 2. These multivariate factors may lead to non-uniform expansion patterns, in particular since the influence of the factors may vary considerably throughout the deployment of the heart valve prosthesis 2. As shown in Fig. 2E the distal end 24 may expand to a greater extent than a proximal end 23 or vice versa as shown in Fig. 2F. This may lead to a geometric center of the heart valve prosthesis 2 being displaced with respect to a balloon catheter and/or the anatomic structure.

The effects of a variation in anatomy of the anatomic structure, in particular the aortic annulus, the left ventricular outflow tract, sinotubular junction, and/or a target implantation depth on the expansion pattern of the heart valve prosthesis 2 may be included in the model. This allows to adapt the medical apparatus for generating control commands to establish the predetermined position/orientation of the heart valve prosthesis with respect to a shape of the anatomic structure. The heart valve prosthesis 2 can for example have a funnel/conical shape which may be particularly advantageous for treatment of an anatomic structure formed by bicuspid aortic valve having a funnel-shaped anatomy.

Figures 2G and 2H show a cross-sectional view of an anatomic structure 9 to which the heart valve prosthesis 2 in a fully deployed configuration comprising a differently shaped proximal and distal sections 23, 24 may conform.

The distal section 24 has a tapered shape. As shown in Fig. 2G and 2H, the convex shape of the distal section 24 may have a varying curvature dependent on the type of the valve and/or the anatomic structure of the patient. The method/medical apparatus is adapted to monitor different funnel/conical shapes as displayed in Figs. 2E and 2F, e.g. by monitoring a plurality of radial dimension along the longitudinal axis of the heart valve prosthesis 2. Based on the different funnel/conical shapes of a specific heart valve prosthesis 2 with respect to a specific anatomic shape, the expected position, orientation, and force exerted on the heart valve prosthesis 2 at a final deployment configuration is determined.

Figure 3 shows a schematic illustration of the medical apparatus 101 comprising a robotic controller 201 for moving and deploying the heart valve prosthesis 2' based on an image sequence intra-operatively in real-time.

The medical apparatus 101 comprises a processing unit 21 which is connected the robotic controller 201 for bidirectional transmission of sensor data from the robotic controller 201 to the processing unit 21 and control commands to the robotic controller 201. The sensor data may exemplarily comprise force data exerted on the medical instruments for placing the heart valve prosthesis 2' intra-operatively.

The medical apparatus 101 is further connected to an imaging device formed by a C-arm 20 for receiving two-dimensional fluoroscopy images throughout deployment of the heart valve prosthesis 2' within an aortic valve of the patient. An adaptive generative model of the medical apparatus 101 was trained using a computer vision algorithm to extract the previously described instrument and anatomic characteristic based on a plurality of previous training fluoroscopy images. The resulting adaptive generative model is configured for predicting the deployment behavior of the heart valve prosthesis 2' intra-operatively based on the intra-operative fluoroscopy data of the C-arm 20.

This predicted deployment is used for determining a plurality of control commands for a robotic controller 201 for positioning and deploying the heart valve prosthesis 2' intra-operatively. This can be achieved by movement control commands for the robotic controller 201 to adjust the position of the heart valve prosthesis 2' along a curvilinear direction, a lateral direction, and/or a tilt of the heart valve prosthesis 2' and to deploy the heart valve prosthesis 2'. The intra-operative deployment of the heart valve prosthesis 2' is carried out by moving the heart valve prosthesis 2' to at least one intermediate deployment configuration as previously described and intermittently determining new control instructions. The final step of converting from one of the intermediate deployment configurations to the final deployment configuration is carried out based on a single control command to preempt prolonged influence of additional multivariant influencing factors, in particular due to the cardiac vasculature interaction with the heart valve prosthesis 2' during a final deployment step.

The processing unit 21 in Fig. 3 is further connected to a display 22 which displays an intra-operative fluoroscopy image 6' in real-time. The fluoroscopy image 6' displays the heart valve prosthesis 2' being deployed by a balloon of a balloon catheter 31'. This enables clinicians to continuously monitor the fully or partially automated positioning, orienting, and deployment of the heart valve prosthesis 2' via the medical apparatus 101, allowing for precise and timely intervention, if necessary. The medical apparatus 101 is adapted for switching to a remote operation or manual operation of the medical apparatus 101 by the clinician.

Alternatively or additionally to sending control commands to the robotic controller 201, the medical apparatus 101 is adapted to provide instructions for carrying out the control command. These instructions may be displayed on a display 22 connected to the medical apparatus 101 such that a deployment system/the heart valve prosthesis 2' can be positioned/oriented and deployed accordingly by a clinician (not shown in Fig. 3).

Figure 4A shows a graph showing a mean, a median, and a standard deviation of a length of the heart valve prosthesis changing throughout the images acquired during the deployment within 147 patients during a transcatheter aortic valve implantation procedure. Figure 4B shows a subsection indicated by the rectangular frame of the graph of Fig. 4A corresponding only to the deployment process of the heart valve prosthesis.

The figures 4A and 4B display the mean as a dashed line and the median as a continuous line. The mean plus/minus the standard deviation is displayed as a grey area in Figs. 4A and 4B.

As is apparent from Figs. 4A and 4B, the mean length of the heart valve prosthesis of the 147 patients decreased during deployment due to the heart valve prosthesis radially expanding/being expanded during deployment. Thus, the length of the heart valve prosthesis in Figs. 4A and 4B decreases from approximately 35 mm to below 27.5 mm.

The corresponding fluoroscopic image sequence of the patients, e.g. the 147 patients, which shows the deployment process, i.e. more or less the rectangular range of Fig. 4A, may be used for training the machine learning model such that only the deployment of the heart valve prosthesis can be accurately modelled. The image sequences may be pre-processed by cutting the fluoroscopic image sequences to only include the deployment and/or cropped to only include a subsection of the fluoroscopic image frames which is relevant in view of the deployment of the heart valve prosthesis.

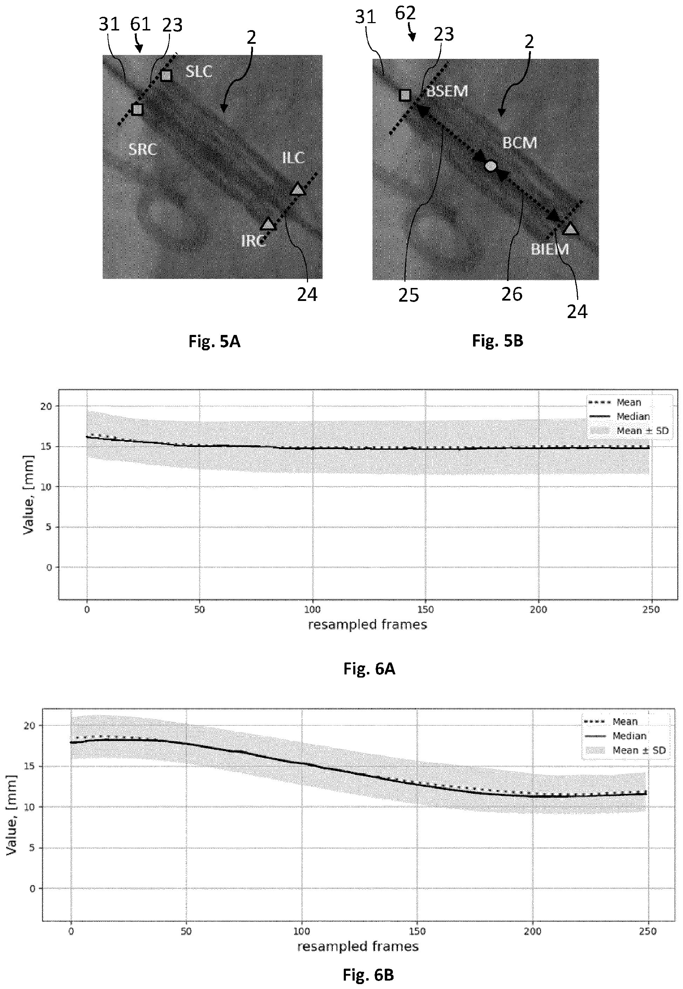

Figures 5A and 5B show a fluoroscopy image 61 showing the superior edge 23 and an inferior edge 24 of the heart valve prosthesis 2 and a fluoroscopy image 62 showing a balloon superior edge marker (BSEM) and balloon inferior edge marker (BIEM) and a balloon center marker (BCM). The heart valve prosthesis 2 in Figs. 5A and 5B is mounted on a balloon catheter 31 and assumes a non-deployed configuration.

Figure 5A shows that the superior edge 23 is determined based on a line leading through a superior left corner SLC and a superior right corner SRC of the heart valve prosthesis 2 shown by two rectangular shapes. The inferior edge 24 is instead determined by a line leading through the inferior left corner ILC and the inferior right corner IRC denoted by the triangular shapes. The corners of the heart valve prosthesis 2 can be reliably tracked by being radiopaque. The distance between the superior edge 23 and the inferior edge 24 of the heart valve prosthesis denoted as doted lines in Fig. 5A decreases during the deployment of the heart valve prosthesis 2 due to the radial expansion.

The fluoroscopy image 62 of Fig. 5B shows a superior distance 25 between the balloon superior edge marker indicated by a rectangular shape and the balloon center marker BCM indicated by a circular shape as a dashed line. Fig. 5B further shows an inferior distance 26 between the balloon inferior edge marker BIEM indicated by a triangular shape and the balloon center marker BCM as a dashed line. The balloon superior edge marker BSEM (Fig. 5B, rectangle) and the balloon inferior edge marker BIEM (Fig. 5B, triangle) may be provided to train/model the deployment model. The balloon superior edge marker and the balloon inferior edge marker may be located at a predetermined distance from the balloon center marker BCM, and preferably essentially are arranged in proximity to the position of the superior and inferior edge 23, 24 of the heart valve prosthesis in a non-deployed configuration.

The distance 25, 26 from an intersection of the line between BSEM and BIEM with the respective superior and inferior edge 23, 24 to the BCM may be used for training the deployment model or computationally model the deployment model.

These changing distances 25, 26 may be used to model the relative displacement of the heart valve prosthesis 2 with respect to the balloon catheter 31. Alternatively, to the distances 25, 26 to the balloon center marker BCM, a distance of a geometric center of the heart valve prosthesis 2 to the balloon center marker BCM may be used to model the deployment behaviour of the heart valve prosthesis 2.

The balloon catheter 31 may comprise radiopaque markers in addition to the balloon center marker BCM which may simplify determining the displacement of the balloon with respect to the heart valve prosthesis 2, in particular with respect to the superior and inferior edge 23, 24. In particular, the balloon catheter 31 may comprise a non-virtual radiopaque balloon superior edge marker BSEM and/or a non-virtual radiopaque balloon inferior edge marker BIEM which simplifies providing these balloon markers to train/model the deployment model.

These distances 25, 26, the superior and inferior edge 23, 24 and/or the different markers SRC, SLC, ILC, IRC may be provided as heart valve prosthesis assembly characteristics. Alternatively or additionally, the heart valve prosthesis assembly characteristic may include a cell size/shape of a stent of the heart valve prosthesis 2 and/or at least a first radial dimension of the heart valve prosthesis 2, in particular a first and second radial dimension of the heart valve prosthesis 2 which are longitudinally spaced apart from each other.

The heart valve prosthesis assembly characteristic may be labelled in the baseline image data with which the model is trained. The heart valve prosthesis assembly characteristic may be extracted using a computer vision algorithm. This may include processing the information of the computer vision algorithm to arrive at the heart valve prosthesis assembly characteristic. Training the model with this heart valve prosthesis assembly characteristic,. in particular by labelled baseline image data, may enable to predict an intra-operative future deployment state having regard to a non-uniform expansion pattern of the heart valve prosthesis.

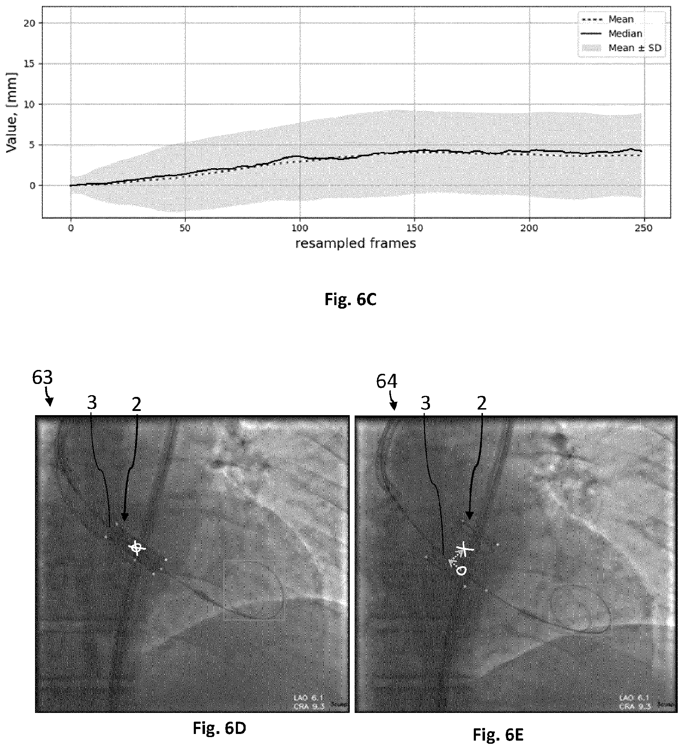

Figure 6A shows a mean, a median, and a standard deviation of the distance from a superior edge of the transcatheter aortic valve to a balloon center marker for the 147 patients. Figure 6B shows a mean, a median, and a standard deviation of a distance from an inferior edge of the transcatheter aortic valve to a balloon center marker for the 147 patients. Figure 6C shows a mean, a median, and a standard deviation of a transcatheter aortic valve centroid shift. Similarly, as shown in Figs. 4A and 4B, the mean is displayed as a dashed line, the median as a continuous line and the standard deviation with respect to the mean is displayed as a grey area. The data shown in Figs. 6A -6C was acquired during a TAVI procedure of the 147 patients.

Figure 6A shows that the distance of the superior edge of the valve prosthesis to the balloon center marker (see Figs. 5A and 5B) changes only marginally on average throughout the deployment procedure of the heart valve prosthesis. In contrast to that, Figure 6B shows that the distance of the inferior edge of the heart valve prosthesis with respect to the balloon center marker changes by approximately 5 mm. As shown in Fig. 6C, the shift of the heart valve prosthesis centroid during the deployment in a TAVI procedure shifts approximately about 5 mm which is almost in its entirety due to the changing distance of the inferior edge of the heart valve prosthesis to the center of the balloon of the balloon catheter. Hence, the relative position of the heart valve prosthesis changes with respect to the deployment instrument formed by the balloon catheter by being displaced in a distal direction away from the heart with respect to the balloon catheter. The position/orientation of the deployment instrument is however essentially constant with respect to the anatomic structure. Therefore, the heart valve prosthesis is misaligned considerably if the position/orientation of the heart valve prosthesis assembly is not preamptively positioned in anticipation of this displacement. The computer implemented method of predicting and the apparatus for predicting the intra-operative future deployment state in which a geometric center of the heart valve prosthesis is displaced accordingly in an axial direction of the heart valve prosthesis assembly can support a clinician/a robotic controller to accurately place the heart valve prosthesis.

Figures 6D and 6E show a first fluoroscopy image 63 of the of the heart valve prosthesis assembly comprising a heart valve prosthesis 2 in a non-deployed state and a second fluoroscopy image 64 of the heart valve prosthesis assembly in a fully deployed state. Figs. 6D and 6E schematically illustrate the effect described in Figs. 1A to 1C regarding the radial displacement of a heart valve prosthesis 2 and Figs. 6A - 6C regarding the axial displacement of the heart valve prosthesis during deployment. The balloon catheter 31 in Figs. 6D and 6E is used as a deployment instrument of the heart valve prosthesis assembly.

Figure 6D shows a geometric center of the heart valve prosthesis 2 indicated by a cross being essentially aligned on a center of a balloon of the balloon catheter 31 which is indicated by a hollow circle. The center of the balloon may be determined based on at least one radiopaque marker (see Figs. 5A and 5B).

Figure 6E shows that the geometric center of the heart valve prosthesis 2 is displaced in the axial direction of the heart valve prosthesis assembly and the radial direction of the heart valve prosthesis assembly as indicated by the two dashed arrows. The geometric center is specifically displaced in a distal axial direction away from the aortic heart valve when carrying out the TAVI procedure shown in Figs. 6D and 6E. Hence, the geometric center marked as a cross is displaced considerably in a non-uniform manner with respect to the geometric center marked as the hollow circle. A deployment model of the computer-implemented method/medical apparatus is configured for modelling the intra-operative future deployment state, e.g. a fully deployed state of the heart valve prosthesis 2 shown in Fig. 6E, based on the current deployment state of the heart valve prosthesis 2, e.g. the non-deployed state shown in Fig. 6D. The intra-operative future deployment state comprises the position of the geometric center of the heart valve prosthesis which is corrected for this radial and axial displacement.

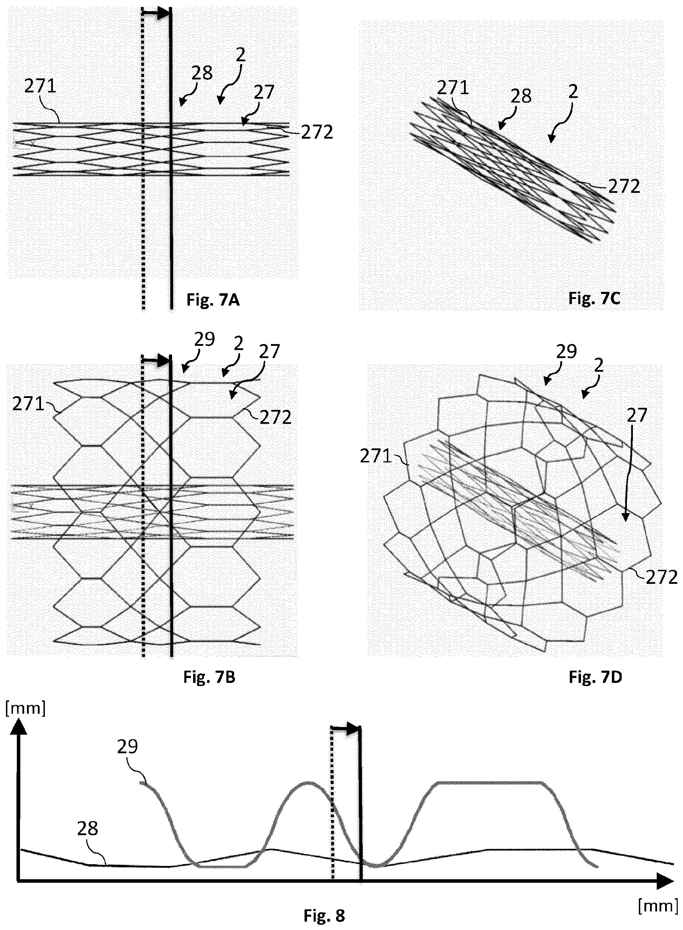

Figures 7A and 7B show a side view of a heart valve prosthesis 2 in a non-deployed configuration and a fully deployed configuration comprising varying open cell sizes 27. Figures 7C and 7D show a perspective side view of the heart valve prosthesis 2 of Figs. 7A and 7B in the non-deployed configuration 28 and the fully deployed configuration 29.

A stent of the heart valve prosthesis 2 includes a plurality of open cells 271, 272 which comprise of a plurality of struts which partially extend along a longitudinal direction and partially extend in a circumferential direction of the heart valve prosthesis 2.

At least some of the struts are essentially parallel to a longitudinal axis of the heart valve prosthesis 2. At least some other struts are inclined with respect to the longitudinal axis.

In the non-deployed configuration 28, these other struts forming the open cells 271, 272 have a circumferential extension and a longitudinal extension (see Figs. 7A and 7C).

In the fully deployed configuration 29, the circumferential extension of the struts increases while the longitudinal extension decreases, i.e. the inclination of the struts with respect to the longitudinal axis increases. Thereby, regions of the stent of the heart valve prosthesis 2 which have more circumferentially extending struts shrink to a greater degree during deployment of the heart valve prosthesis 2. This leads to an uneven expansion of the heart valve prosthesis 2, in particular an uneven expansion of the heart valve prosthesis 2 during conversion from the non-deployed configuration 28 to the fully deployed configuration 29. This uneven expansion may occur for balloon-deployable heart valve prostheses 2 or self-expanding heart valve prostheses 2.

During conversion of the heart valve prosthesis 2 from the non-deployed configuration 28 to the deployed configuration 29, a plurality of these struts increases in inclination with respect to the longitudinal axis, leading to local shrinkage. On the other hand, a plurality of struts which only extend in the longitudinal direction retain essentially the same longitudinal extension, i.e. remain parallel to the longitudinal axis (see Figs. 7A - 7D).

This uneven self-expansion can be measured by monitoring the cell size 27 of different open cells 271, 272. For example, a first open cell 271 may which has fewer/shorter longitudinal struts may decrease in length during deployment to a greater degree than a second open cell 272 which has more/larger longitudinal struts. Alternatively, to the open cell size 27, a circumferential and/or longitudinal extension of the open cells 271, 272, and/or an inclination of the struts may be used for training or computationally modelling the deployment model.

This deployment model accounting for the uneven expansion may be used to predict an intra-operative future deployment state of the heart valve prosthesis 2, i.e. a different shrinkage of sections of the heart valve prosthesis 2 in an axial direction. As previously described, the heart valve prosthesis typically longitudinally shrinks during deployment in a direction away from the heart of a patient, i.e. such that the geometric center in the non-deployed configuration 28 indicated by the dashed line in Figs. 7A and 7B is displaced in the fully deployed configuration 29 indicated by the solid line.

The deployment model to predict the intra-operative future deployment state may include a three-dimensional finite element model which may model this uneven expansion, i.e. the different shrinkage of the heart valve prosthesis 2 around its center of mass during deployment.

Figure 8 shows a graph of the average circumferential width of the open cells of a stent of the heart valve prosthesis 2 with respect to the longitudinal position of the stent of the non-deployed and fully deployed configuration of Figs. 7A to 7D. The circumferential width of the heart valve prosthesis 2 in the non-deployed configuration 28 is shown as a black line and the circumferential width of the heart valve prosthesis 2 in the fully deployed configuration 29 is shown as a grey line.

The open cells (see Figs. 7A - 7D) which have more/larger longitudinal struts shrink less and the open cells which have fewer/smaller longitudinal struts shrink more. This leads to the uneven expansion, i.e. the geometric center of the heart valve prosthesis 2 in the non-deployed configuration 28 indicated by the dashed line moves relative to the geometric center of the heart valve prosthesis 2 in the fully deployed configuration 29 indicated by the solid line.

## Claims

1. A computer-implemented method for training at least one deployment model to predict a deployment of a heart valve prosthesis assembly (2, 3, 4) comprising the steps of:
- Receiving, by one or more processors, a baseline image sequence, wherein the baseline image sequence comprises images which include the heart valve prosthesis assembly (2, 3, 4) within a native anatomic structure (9) in at least two different deployment states,
- Extracting at least one heart valve prosthesis assembly characteristic of the heart valve prosthesis assembly (2, 3, 4) from the baseline image sequence,
- Optionally extracting at least one anatomic characteristic of the native anatomic structure (9) from the baseline image sequence,
- training the at least one deployment model for the heart valve prosthesis assembly (2, 3, 4) based on the heart valve prosthesis assembly characteristic, and optionally the anatomic characteristic, the deployment model being configured for predicting an intra-operative future deployment state of a heart valve prosthesis assembly (2', 3', 4') based on an intra-operative image sequence comprising the heart valve prosthesis assembly (2', 3', 4') in a current deployment state.

2. The method of claim 1, wherein the heart valve prosthesis assembly characteristic comprises at least one of:
(i) A specific heart valve prosthesis type,
(ii) A heart valve prosthesis dimension, in particular at least one radial dimension (R) and/or longitudinal dimension (L), preferably a first radial dimension at at least a first longitudinal location of the heart valve prosthesis (2) and a second radial dimension at at least a second longitudinal location of the heart valve prosthesis (2) different than the first longitudinal location,
(iii) A point-to-point or line-to-line distance between the heart valve prosthesis (2) and the anatomic structure (9) or a medical instrument, in particular a medical instrument formed by a catheter and/or guidewire having a pre-defined shape, and/or
(iv) Deployment dynamics of deploying the heart valve prosthesis (2), in particular a relation between time for deployment, a velocity of deployment, and/or an acceleration of deployment, and/or
(v) A metric indicative of a displacement of a geometric center of the heart valve prosthesis (2') relative to a deployment instrument (3') or the anatomic structure (9) in an axial direction of the heart valve prosthesis assembly (2', 3', 4').

3. The method of the claims 1 to 2, wherein the anatomic characteristic is selected from at least one of:
(i) An aortic annulus radial dimension (11), an aortic root radial dimension, a sinotubular junction radial dimension, and/or a left ventricular outflow tract radial dimension (10),
(ii) A two- or three-dimensional anatomic shape of the anatomic structure (9), in particular including changes to the anatomic shape due to a respiratory or cardiac rhythm,
(iii) A blood pressure measurement,
(iv) A blood flow measurement,
(v) A respiratory and/or cardiac rhythm measurement, and
(vi) Alterations of the anatomic structure (9) due to an external factor, in particular an application of rapid pacing, preferably a frequency and/or duration of the rapid pacing application.

4. The method according to one of the preceding claims, wherein the heart valve prosthesis assembly characteristic, and optionally the anatomic characteristic, are monitored for different deployment stages included in the baseline image sequence including the steps of:
(i) Monitoring a position and/or an orientation of the heart valve prosthesis (2) with respect to a deployment instrument (3),
(ii) Monitoring a position and/or orientation of a medical navigation and/or deployment instrument (3) functionally delivering and/or deploying the heart valve prosthesis (2) with respect to the anatomic characteristic, in particular the two- and or three-dimensional anatomic shape, the aortic radial dimension, the aortic root radial dimension, the sinotubular junction radial dimension, and/or the left ventricular outflow tract radial dimension, and/or
(iii) Adjusting the heart valve prosthesis assembly characteristic, and optionally the anatomic characteristic, to correct for an obstruction of view and/or a parallax phenomenon, if any is detected during the different deployment stages.

5. The method of one of the preceding claims, wherein the deployment model is configured for predicting the intra-operative future deployment state taking into account at least one of:
(i) a relationship between the change in length of the heart valve prosthesis with a radial expansion of the heart valve prosthesis,
(ii) a relationship between the type and/or size of the heart valve prosthesis (2) and a predicted radial expansion and/or change in length of the heart valve prosthesis (2),
(iii) a movement of a geometric center of the heart valve prosthesis (2) and/or a change in orientation of the heart valve prosthesis (2), and
(iv) a non-uniform expansion pattern of the heart valve prosthesis (2) during the different deployment stages, in particular based on a change of a first radial dimension at a distal location in relation to a change of a second radial dimension at a proximal location of the heart valve prosthesis (2) or on a varying open cell size (27) of the heart valve prosthesis (2).

6. The method of one of the preceding claims, wherein the deployment model is a biomechanical model which includes the two- or three-dimensional anatomic shape and is configured to simulate and/or evaluate the deployment of the heart valve prosthesis (2) within the anatomic structure (9), wherein the deployment model is selected from at least one of:
(i) a quasi-static deployment model,
(ii) a two-dimensional finite element model, optionally comprising a plurality of beam elements,
(iii) a three-dimensional finite element model,
(iv) a dynamic finite element model, which is preferably adapted for determining forces between the heart valve prosthesis and the anatomic shape during deployment, and
(v) a computational fluid dynamics model, in particular for predicting valve regurgitation for the deployed heart valve prosthesis (2).

7. The method according to claim 6, wherein the anatomic shape comprises a radial dimension or shape of the aortic valve annulus, a radial dimension or shape of the left ventricular outflow tract, and/or a radial dimension or shape of the aortic root, and wherein
the deployment model is configured for modelling the position, shape, and/or force on the anatomic shape or a fully deployed heart valve prosthesis (2) having a cylindrical, conical, or funnel shape within the anatomic shape.

8. The method of one of the preceding claims, wherein a plurality of deployment models is determined independently for subgroups of the baseline image sequence, subgroups of the heart valve prosthesis assembly characteristic, and/or subgroups of the anatomic characteristic.

9. The method of claim 8, wherein an adaptive generative model is adapted for predicting the deployment of the heart valve prosthesis assembly (2'; 3'; 4') based on the deployment model of the plurality of deployment models and based on a threshold similarity of the baseline image sequence, the heart valve prosthesis assembly characteristic, and/or the anatomic characteristic with the corresponding intra-operative image sequence, an intra-operative heart valve prosthesis assembly characteristic, and/or an intra-operative anatomic characteristic.

10. A computer program comprising instructions which, when the program is executed by a computer, cause the computer to carry out the method of one of the claims 1 to 9.

11. A non-transitory computer-readable medium having stored there on a computer program product comprising code which, when executed on a processor carries out the method according to one of the claims 1 - 9.

12. A trained machine-learning model for predicting an intra-operative future deployment state of a heart valve prosthesis assembly (2', 3', 4') based on an intra-operative image sequence comprising the heart valve prosthesis assembly (2', 3', 4') trained according to the claims 1 - 9.

13. Computer-implemented method of predicting a deployment of a heart valve prosthesis assembly (2, 3, 4) comprising the steps of:
- Receiving, by one or more processor, an intra-operative image sequence, wherein the intra-operative image sequence comprises images which include the heart valve prosthesis assembly (2', 3', 4') in a current deployment state,
- Analysing the intra-operative image sequence, at least one image of the intra-operative image sequence including the heart valve prosthesis assembly characteristic of the heart valve prosthesis assembly (2', 3', 4'),
- Predicting an intra-operative future deployment state of the heart valve prosthesis assembly (2', 3', 4') using a deployment model, in particular a trained deployment model according to one of the claims 1 - 9, based on the intra-operative image sequence.

14. The method according to claim 13, wherein the intra-operative future deployment state includes a virtual future geometric center of the heart valve prosthesis assembly which is displaced relative to a deployment instrument (3') in an axial direction of the heart valve prosthesis assembly (2', 3', 4') with respect to a current geometric center of the current deployment state, in particular the virtual future geometric center of the heart valve prosthesis (2') is displaced in a proximal axial direction with respect to the current geometric center of the current deployment state.

15. The computer-implemented method according to claim 13 or 14, wherein the method includes determining the output data including at least one of:
- display data for displaying the intra-operative future deployment state on a display (22),
- display data of an initial intra-operative deployment state of the heart valve prosthesis assembly (2) to achieve the intra-operative future deployment state through deployment from the initial intra-operative deployment state on a display (22),
- display data for displaying user instructions to achieve the intra-operative future deployment state on the display (22),
- including risk data indicative of a risk assessment of the intra-operative future deployment state, in particular with respect to the anatomic structure, and
- including deployment parameters for achieving the intra-operative future deployment state.

16. A medical apparatus (101) for positioning and deploying a heart valve prosthesis assembly (2', 3', 4') comprising:
- An input interface for receiving an intra-operative image sequence, in particular an intra-operative fluoroscopy image sequence, and in particular a pre-operative computer tomography image sequence,
- A processing unit (21) comprising an interface for accessing the deployment model or comprising the deployment model of one of the claims 12 - 14, in particular generated by the computer-implemented method according to one of the claims 1 to 9,
- The processing unit (21) being adapted for predicting an intra-operative future deployment state of the heart valve prosthesis assembly (2', 3', 4') based on the deployment model and the intra-operative image sequence comprising the heart valve prosthesis assembly (2', 3', 4') in a current deployment state,
- The processing unit (21) being adapted for generating output data comprising at least one control instruction for deploying and/or moving the heart valve prosthesis assembly (2', 3', 4') based on the predicted intra-operative future deployment state, wherein the control instruction is either a control command or display data comprising user instructions for being displayed on a display for achieving the intra-operative future deployment state in real-time,
- An output interface for transmitting the at least one output data to a robotic controller (201) adapted for carrying out the control command and/or to the display (22).

17. The medical apparatus (101) according to claim 16, wherein the processing unit (21) is adapted for tracking a heart valve prosthesis (2) of the heart valve prosthesis assembly (2, 3, 4), in particular a terminal proximal end of the heart valve prosthesis (2) and/or a terminal distal end of the heart valve prosthesis (2) based on detecting a radiopaque section of the heart valve prosthesis (2) in the intra-operative image sequence.

18. The medical apparatus (101) according to one of the claims 16 or 17, wherein the processing unit (21) is adapted for tracking a deployment instrument (3) and/or a navigation instrument (4) of the heart valve prosthesis assembly (2, 3, 4), in particular a center location of the deployment instrument (3), based on detecting at least one radiopaque section of the deployment instrument (3) and/or the navigation instrument (4).

19. The medical apparatus (101) according to one of the claims 16 to 18, wherein the processing unit (21) is adapted for determining at least one inflation control command for a partial inflation of a balloon of a balloon catheter for deploying the heart valve prosthesis (2') to an intermediate deployment configuration, in particular such that the balloon of the balloon catheter (31') is inflated between 50% and 90%, preferably between 65% and 85%, in particular between 70% and 80%, with respect to a final deployed configuration of the balloon and, wherein
the processing unit (21) is adapted for thereafter determining at least one intermediate positioning control command for repositioning of the heart valve prosthesis (2') with respect to the anatomic structure (9).

20. The medical apparatus (101) according to one of the claims 16 to 19, wherein the medical apparatus (101) comprises the robotic controller (201) for operating and moving the medical instrument for deployment of the heart valve prosthesis.

21. The medical apparatus (101) according to claim 16 to 20, wherein the at least one control command is configured for operating the robotic controller (201) to deploy and/or move the heart valve prosthesis (2), in particular to move the heart valve prosthesis (2) in a longitudinal direction, a lateral direction, and/or to tilt the heart valve prosthesis (2) or the medical deployment/navigation instrument (3; 4).

22. The medical apparatus (101) according to one of the claims 16 to 21, wherein the processing unit is adapted for predicting the deployment of the intra-operative heart valve prosthesis assembly (2'; 3'; 4') based on sensor data, in particular sensor data receivable from the robotic controller (201), wherein
the processing unit (21) is configured for applying a sensor fusion algorithm, in particular a Kalman Filter algorithm, to the sensor data and data generated by the deployment model to generate the control command.

23. Method for positioning and deploying a heart valve prosthesis assembly (2), comprising:
- receiving an intra-operative image sequence, in particular an intra-operative fluoroscopy image sequence, and preferably a pre-operative computer tomography image sequence,
- predicting an intra-operative deployment state of the heart valve prosthesis assembly (2', 3', 4') comprising a heart valve prosthesis (2') during different deployment stages of the heart valve prosthesis (2') based on the intra-operative image sequence and a deployment model, in particular generated by the computer implemented method according to one of the claims 1 to 9,
- generating output data comprising at least one control instruction for deploying and/or moving the heart valve prosthesis (2') based on the predicted intra-operative future deployment state, wherein the control instruction is either a control command or display data comprising user instructions to be displayed on a display (22) for achieving the intra-operative future deployment state in real-time,
- transmitting the at least one output data to the display (22) and/or a robotic controller (201) adapted for positioning and deploying the heart valve prosthesis (2') to the intra-operative future deployment state,
- deploying and/or positioning the heart valve prosthesis (2), in particular moving the heart valve prosthesis (2) in a longitudinal direction and/or tilting the heart valve prosthesis (2) or the medical deployment/navigation instrument (3; 4) in a partially or fully automated manner by the robotic controller (201) or manually based on instructions on the display (22).
